# FASCICULE DE BREVET EUROPEEN

(11) **EP 2 442 767 B1**
(45) Date de publication et mention de la délivrance du brevet: **17.02.2016**
(21) Numéro de dépôt: 10738005.7
(22) Date de dépôt: 14.06.2010
(51) Int. Cl.: A61F 6/14

(54) **STÉRILET**
INTRAUTERINE VORRICHTUNG
INTRAUTERINE DEVICE

(30) Priorité: 16.06.2009 FR 0902918
(43) Date de publication de la demande: 25.04.2012
(73) Titulaire: CL Investissements, 03700 Serbannes (FR)
(72) Inventeur: LAFONT, Charles-Dominique, 03700 Serbannes (FR)
(74) Mandataire: Myon, Gérard Jean-Pierre
(86) Numéro de dépôt international: PCT/FR2010/051181
(87) Numéro de publication internationale: WO 2010/146295

(56) Documents cités:
- WO-A-88/09648
- DE-A1-102004 061 823
- FR-A- 2 555 893
- GB-A- 2 079 158
- GB-A- 2 121 289
- US-A- 3 993 058

## Description

L'invention a trait à un stérilet.

Parmi les dispositifs à usage contraceptif, il est connu d'utiliser des stérilets contenant du cuivre. Ce type de dispositif est également désigné par l'expression DIU ou Dispositif Intra Utérin. Ces stérilets sont d'une fabrication aisée, peu onéreuse tout en ayant une efficacité reconnue, d'où leur emploi depuis de nombreuses années.

Ce type de stérilet comprend un corps, configuré en T, constitué de deux bras, flexibles, s'étendant à partir d'une extrémité d'une tige. Les bras sont légèrement cintrés en direction de l'extrémité libre de la tige, de manière à donner au stérilet l'aspect d'une ancre de marine. Par effet ressort, les bras assurent le maintien en position du stérilet dans l'utérus, du fait de leur flexibilité.

Un fil de cuivre est enroulé en spirale autour de la tige. Le stérilet agit par effet mécanique et par effet biochimique. En effet, la présence du stérilet modifie la configuration de l'utérus et le cuivre, par action des ions cuivriques, altère la fonction et la viabilité des gamètes mâles et femelles.

Dans la mesure où les bras du stérilet se trouvent au plus près de la paroi de l'utérus et des trompes de Fallope, il est intéressant de disposer le principe actif chimique, à savoir le cuivre, au niveau des bras. Pour cela, on connaît des stérilets, tels ceux initialement commercialisés sous l'appellation de GYNE T380 par la société CILAG dont la tige est recouverte d'un fil de cuivre, aux spires lâches, et dont les extrémités des bras sont pourvues d'un manchon de cuivre. Ainsi, la surface d'échange, pour la diffusion des ions cuivriques, d'un tel stérilet est d'environ 380 mm², c'est-à-dire comparable à celle d'un stérilet dont seule la tige est pourvue d'un fil de cuivre, enroulé en spires serrées. Il est en effet préconisé, selon la norme internationale ISO 7439, une surface d'échange comprise entre 200 et 380 mm². Une telle surface correspond à une efficacité optimale concernant l'action des ions cuivriques sur les gamètes.

Un tel stérilet, avec des manchons de cuivre sur les bras, outre le fait que la surface d'échange est mieux répartie sur l'ensemble du stérilet, permet de placer le cuivre au plus près de la paroi de l'utérus et des trompes de Fallope, lorsque le stérilet est en place. En revanche, les bras sont plus rigides, du fait du manchon en cuivre. Ceci ne facilite pas la mise en place du stérilet et peut s'avérer douloureux lors de son retrait.

GB-A-2 079 158 décrit un stérilet en forme d'étoile à trois branches souples. Les branches sont en polymère, recouvertes d'une couche externe active comportant un silicone, une hormone et des traces d'éléments minéraux, tels le cuivre.

US-A-3 993 058 concerne un stérilet configuré en T. Le stérilet est creux et forme un réservoir contenant un agent actif, par exemple une hormone. Les parois du réservoir sont en matériau perméable. Le stérilet peut également comprendre un manchon en cuivre.

Ces stérilets ne permettent pas une diffusion optimale du ou des agents actifs.

C'est à ces inconvénients qu'entend plus particulièrement remédier l'invention en proposant un stérilet avec une surface d'échange optimisée, souple, aisé à mettre en place et à retirer.

A cet effet, l'invention a pour objet un stérilet comprenant un corps configuré globalement en forme de T comportant deux bras, flexibles, s'étendant à partir d'une extrémité d'une tige, au moins une couche externe d'une partie des bras étant pourvue d'un matériau contenant au moins du silicone et une poudre de cuivre, caractérisé en ce que le matériau comprend entre 20 % et 60 % en poids de silicone et de poudre de cuivre pur.

Ainsi, avec un matériau constitutif d'une partie des bras du stérilet comportant de la poudre de cuivre et du silicone, on assure une contraception efficace, par libération d'ions cuivriques au plus prés de la paroi de l'utérus et des trompes de Fallope, tout en maintenant la flexibilité des bras du stérilet, d'où une mise en place et un retrait aisés.

Selon des aspects avantageux mais non obligatoires de l'invention, un tel stérilet peut incorporer une ou plusieurs des caractéristiques suivantes :
- Le matériau comprend 50 % en poids de silicone et 50 % en poids de poudre de cuivre pur.
- Le corps du stérilet est entièrement recouvert par une couche de matériau contenant au moins le silicone et la poudre de cuivre.
- La tige est revêtue du même matériau que les bras.
- La couche de matériau a une épaisseur comprise entre 0,2 et 0,4 millimètre.
- Le corps du stérilet est entièrement réalisé dans le matériau contenant au moins le silicone et la poudre de cuivre.
- La poudre de cuivre est une poudre de cuivre d'une pureté égale ou supérieure à 99,9% et d'une granulométrie inférieure ou égale à 10 µm.
- Une hormone progestative est incorporée au matériau.
- Le stérilet est couvert d'une membrane poreuse adaptée pour permettre une libération régulière et contrôlée du principe actif.

L'invention sera mieux comprise et d'autres avantages de celle-ci apparaîtront plus clairement à la lecture de la description qui va suivre d'un stérilet conforme à l'invention, donnée uniquement à titre d'exemple et faite en se référant aux dessins annexés dans lesquels :
- la figure 1 est une vue générale d'un stérilet conforme à l'invention et
- la figure 2 est une coupe, selon le plan II, du stérilet de la figure 1.

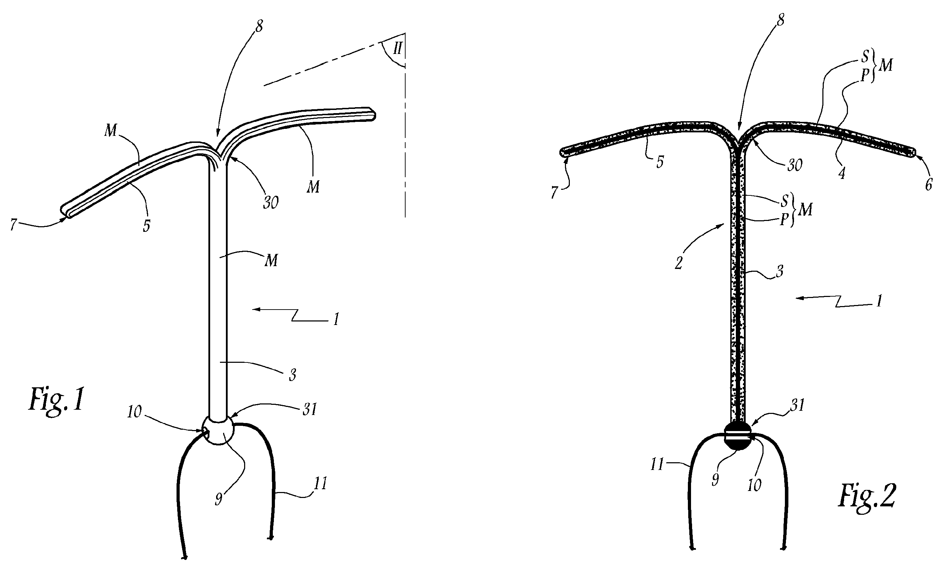

Le stérilet 1, représenté à la figure 1, comprend un corps principal 2. Ce corps 2, visible à la figure 2, est réalisé dans un polymère inerte vis-à-vis des fluides biologiques, c'est à dire biocompatible. Avantageusement, il s'agit d'un polyéthylène de qualité médicale. Le corps 2 forme une structure constitutive du stérilet 1. En d'autres termes, le corps 2 assure le maintien de la forme du stérilet 1.

Ce corps 2 est configuré en T. Il comprend une tige 3, équipée à une extrémité 30 de deux bras 4 et 5. Ces bras 4, 5 s'étendent perpendiculairement de part et d'autre de l'extrémité 30. Ils sont légèrement cintrés en direction de la tige 3 et confèrent au corps 2 une forme d'ancre de marine ou d'hameçon double.

Les extrémités 6 et 7 libres des bras 4 et 5 sont arrondies afin d'éviter tout risque de blessure lorsque le stérilet 1 est en place. La zone de jonction 8 des bras 4 et 5 avec l'extrémité 30 de la tige 3 est globalement configurée en V. En fait, comme illustré à la figure 2, la zone de jonction 8 est obtenue par séparation en deux de la tige 3.

L'extrémité libre 31 de cette tige 3 est pourvue d'un bossage 9 dans lequel un orifice traversant 10 est ménagé. Cet orifice 10 permet le passage d'un fil 11 assurant la mise en place et le retrait du stérilet 1 par un praticien.

Au moins une couche externe d'une partie des bras 4 et 5 est revêtue d'un matériau M souple. En l'espèce, ce matériau M recouvre au moins la surface externe des bras 4 et 5. Avantageusement, comme illustré aux figures 1 et 2, l'ensemble de la surface externe du corps 2 du stérilet 1 est couverte de ce matériau. Ce matériau comprend au moins deux composants. L'un des composants est un silicone S, de qualité médicale, et l'autre composant est une poudre à base de cuivre P. Avantageusement, Il s'agit d'un mélange M avec une répartition homogène de la poudre P à base de cuivre dans le silicone S.

Cette poudre à base de cuivre est, avantageusement, une poudre de cuivre pur P, c'est-à-dire d'une pureté égale ou supérieure à 99,9%. La granulométrie moyenne d'une telle poudre est inférieure ou égale à 10 µm. Cette poudre est connue en soi. Il s'agit, par exemple, de celle commercialisée sous la référence MT 100 par la société MC METAUX & CHIMIE.

La proportion de chacun des composants du matériau M varie entre 30 et 60% en poids. Dans un autre mode de réalisation le matériau est composé en poids de 50% de silicone S et de 50% de poudre de cuivre pur P.

Le matériau M est réparti, de manière homogène, autour de la tige 3 et des bras 4 et 5 en polyéthylène. L'épaisseur de la couche de matériau M est comprise entre 0,2 et 0,4 mm.

Une telle épaisseur du matériau M sur les bras 4, 5 permet de préserver la flexibilité de ces derniers, cela d'autant que le mélange de silicone S et de poudre de cuivre P est lui-même souple, même après polymérisation du silicone. On facilite ainsi la mise en place et le retrait du stérilet 1.

Du fait de la répartition homogène du matériau M sur la tige 3 et les bras 4 et 5, on optimise la surface d'échange, puisque un tel stérilet présente une surface d'échange supérieure à 380 mm². Une telle surface permet d'obtenir un taux de diffusion des ions cuivriques à travers le matériau M équivalent au taux de diffusion des ions cuivriques dans un stérilet comportant un fil de cuivre avec une surface d'échange de 380 mm².

L'efficacité du stérilet est également optimisée par le fait que non seulement la tige 3 mais également les bras 4 et 5 portent du cuivre ce qui permet une action au plus prés de l'utérus et des trompes de Fallope.

Le cuivre, plus précisément les ions cuivriques, diffusent de manière homogène à travers le silicone S, ce qui permet une libération contrôlée d'environ 60 à 80 µg d'ions cuivriques par jour, pendant au moins 4 ans.

Le silicone S forme ainsi une matrice pour le cuivre. En d'autres termes le silicone se comporte comme une membrane poreuse à travers laquelle diffusent les ions cuivriques, en suivant les lois de FICK sur la diffusion membranaire.

Dans un mode de réalisation non illustré, le matériau M, outre la poudre de cuivre pur P et le silicone S, comprend un autre produit, par exemple une hormone progestative. Dans ce cas, on associe deux principes actifs contraceptifs, à savoir le cuivre et l'hormone, dans un même dispositif de contraception intra-utérin. L'hormone et le cuivre diffusent à travers le silicone, de manière régulière et continue. Cette diffusion est d'environ 60 à 80 µg par jour, pendant au moins 4 ans pour les ions cuivriques, et la diffusion de l'hormone est de l'ordre de 20 µg par jour pendant au moins 4 ans.

En variante, non illustrée, la tige 3 du stérilet 1 est recouverte d'une membrane. Une telle membrane protège le stérilet sans nuire à sa souplesse. Cette membrane est poreuse et permet la diffusion du cuivre, éventuellement d'une hormone progestative lorsque cette dernière est incorporée au matériau. La membrane est avantageusement réalisée dans un silicone de type médical. Par ailleurs, la présence d'une membrane participe à une diffusion régulière et contrôlé du cuivre.

Un tel stérilet, lorsqu'il ne comporte que du cuivre et du silicone, répond à la réglementation applicable en France sur les dispositifs médicaux intra-utérins et il n'est donc pas nécessaire d'obtenir une autorisation de mise sur le marché pour ce produit.

Un tel stérilet 1, tel que représenté aux figures 1 et 2, est avantageusement réalisé par surmoulage, par des techniques connues en soi, du matériau M autour d'un corps 2 réalisé en polyéthylène. Avantageusement ce corps 2 en polyéthylène est celui utilisé actuellement pour les stérilets comportant un fil de cuivre enroulé autour de la seule tige.

Dans un autre mode de réalisation de l'invention, non illustré, l'ensemble du corps 2 du stérilet 1 est réalisé dans le matériau comportant le silicone et la poudre de cuivre. Dans ce cas, le silicone utilisé est différent de celui employé lorsque le matériau est employé pour recouvrir, au moins partiellement, le corps 2 du stérilet 1. En effet, il est nécessaire que le matériau présente une certaine rigidité pour préserver la forme initiale du stérilet, tout en ayant une certaine flexibilité des bras, pour le maintien en place du stérilet. Pour cela, on utilise un ou plusieurs silicones associés dont la viscosité, lorsque le silicone n'est pas polymérisée est, à température ambiante, comprise entre 140 000 et 160 000 mPa.s

Le matériau M est réalisé, au préalable, par mélange homogène d'une poudre de cuivre pur P et d'un silicone S de qualité médicale, par exemple celui commercialisé sous l'appellation MED3-4213 de la société NUSIL.

Ce silicone est à l'état liquide ou, du moins, pâteux. En d'autres termes, il n'est pas polymérisé. Il est ainsi possible de recouvrir le corps 2 d'une épaisseur donnée de matériau M. En variante, le stérilet 1 peut être réalisé par passage du corps 2 dans un bain comportant un mélange, non polymérisé et liquide, à base de poudre de cuivre pur et de silicone. Dans ce cas, la viscosité du silicone non polymérisé utilisé est, à température ambiante, comprise entre 70 000 et 90 000 mPa.s.

Que la fabrication du stérilet se fasse par passage dans un bain ou par surmoulage, la polymérisation du silicone est réalisée ultérieurement, par des méthodes connues en soi, par exemple par polymérisation avec addition d'un catalyseur.

En variante, on peut envisager un stérilet dont les branches et une partie seulement de la tige sont recouvertes de matériau. Dans ce cas, la surface d'échange est moindre que celle mentionnée précédemment. Il convient, pour obtenir une efficacité au niveau de la diffusion du principe actif, d'augmenter sensiblement la quantité de poudre de cuivre pur dans le matériau. A titre d'exemple, le pourcentage, en poids, de poudre de cuivre pur dans le matériau peut aller jusqu'à 60%.

## Revendications

1. Stérilet (1) comprenant un corps (2) configuré globalement en forme de T comportant deux bras (4, 5), flexibles, s'étendant à partir d'une extrémité (30) d'une tige (3), au moins une couche externe d'une partie des bras (4, 5) étant pourvue d'un matériau (M) contenant au moins du silicone (S) et une poudre de cuivre (P), **caractérisé en ce que** le matériau (M) comprend entre 30% et 60% en poids de silicone (S) et de poudre de cuivre pur (P).

2. Stérilet selon la revendication 1, **caractérisé en ce que** le matériau (M) comprend 50% en poids de silicone (S) et 50% en poids de poudre de cuivre pur (P).

3. Stérilet selon l'une des revendications précédentes, **caractérisé en ce que** le corps (2) du stérilet est entièrement recouvert par une couche de matériau (M) contenant au moins le silicone (S) et la poudre de cuivre (P).

4. Stérilet selon l'une des revendications précédentes, **caractérisé en ce que** la tige (3) est revêtue du même matériau (M) que les bras (4, 5).

5. Stérilet selon l'une des revendications précédentes, **caractérisé en ce que** la couche de matériau (M) a une épaisseur comprise entre 0,2 et 0,4 millimètre.

6. Stérilet selon la revendication 1, **caractérisé en ce que** le corps (2) du stérilet est entièrement réalisé dans le matériau (M) contenant au moins le silicone (S) et la poudre de cuivre (P).

7. Stérilet selon l'une des revendications précédentes, **caractérisé en ce que** la poudre de cuivre (P) est une poudre de cuivre pur, d'une pureté égale ou supérieure à 99,9% et d'une granulométrie inférieure ou égale à 10 µm.

8. Stérilet selon l'une des revendications précédentes, **caractérisé en ce qu'**une hormone progestative est incorporée au matériau (M).

9. Stérilet selon l'une des revendications précédentes, **caractérisé en ce que** le matériau (M) est couvert d'une membrane poreuse adaptée pour permettre une libération régulière et contrôlée du principe actif (P).

## Patentansprüche

1. Intrauterine Vorrichtung (1), die einen Körper (2) umfasst, der im Ganzen gesehen in der Form eines Ts ausgebildet ist und zwei flexible Arme aufweist, die sich von einem Ende (30) eines Stabes (3) erstrecken, wobei mindestens eine äußere Schicht eines Teils der Arme (4, 5) mit einem Material (M) ausgestattet ist, das mindestens Silikon (S) und ein Kupferpulver (P) enthält, **dadurch gekennzeichnet, dass** das Material (M) zwischen 30 Gew.-% und 60 Gew.-% Silikon (S) und reines Kupferpulver (P) enthält.

2. Intrauterine Vorrichtung nach Anspruch 1, **dadurch gekennzeichnet, dass** das Material (M) 50 Gew.-% Silikon (S) und 50 Gew.-% reines Kupferpulver (P) enthält.

3. Intrauterine Vorrichtung nach einem der vorhergehenden Ansprüche, **dadurch gekennzeichnet, dass** der Körper (2) der intrauterinen Vorrichtung vollständig von einer Schicht aus mindestens dem das Silikon (S) und das Kupferpulver P) enthaltenden Material (M) bedeckt ist.

4. Intrauterine Vorrichtung nach einem der vorhergehenden Ansprüche, **dadurch gekennzeichnet, dass** der Stab (3) mit demselben Material (M) wie die Arme (4, 5) beschichtet ist.

5. Intrauterine Vorrichtung nach einem der vorhergehenden Ansprüche, **dadurch gekennzeichnet, dass** die Schicht aus dem Material (M) eine Dicke zwischen 0,2 und 0,4 mm aufweist.

6. Intrauterine Vorrichtung nach Anspruch 1, **dadurch gekennzeichnet, dass** der Körper (2) der intrauterinen Vorrichtung vollständig aus dem Material (M) hergestellt ist, das mindestens das Silikon (S) und das Kupferpulver (P) enthält.

7. Intrauterine Vorrichtung nach einem der vorhergehenden Ansprüche, **dadurch gekennzeichnet, dass** das Kupferpulver (P) ein reines Kupferpulver ist mit einer Reinheit gleich oder größer als 99,9% und einer Korngröße kleiner oder gleich 10 µm.

8. Intrauterine Vorrichtung nach einem der vorhergehenden Ansprüche, **dadurch gekennzeichnet, dass** ein Gestagen Hormon in dem Material (M) eingeschlossen ist.

9. Intrauterine Vorrichtung nach einem der vorhergehenden Ansprüche, **dadurch gekennzeichnet, dass** das Material (M) mit einer porösen Membran bedeckt ist, die ausgebildet ist, eine gleichmäßige und kontrollierte Freisetzung des Wirkstoffs (P) zu gestatten.

## Claims

1. Intrauterine device (1) comprising a body (2) generally configured in a T-shape having two flexible arms (4, 5) extending from an end (30) of a stem (3), at least one outer layer of a portion of the arms (4, 5) being provided with a material (M) comprising at least silicone (2) and a copper powder (P), **characterised in that** the material (M) comprises between 30 % and 60 % by weight of silicone (S) and of pure copper powder (P).

2. Intrauterine device according to claim 1, **characterised in that** the material (M) comprises 50 % by weight of silicone (S) and 50 % by weight of pure copper powder (P).

3. Intrauterine device according to one of the preceding claims, **characterised in that** the body (2) of the intrauterine device is entirely covered with a layer of material (M) comprising at least the silicone (S) and the copper powder (P).

4. Intrauterine device according to one of the preceding claims, **characterised in that** the stem (3) is coated with the same material (M) as the arms (4, 5).

5. Intrauterine device according to one of the preceding claims, **characterised in that** the layer of material (M) has a thickness between 0.2 and 0.4 millimetre.

6. Intrauterine device according to claim 1, **characterised in that** the body (2) of the intrauterine device is made entirely from the material (M) comprising at least the silicone (S) and the copper powder (P).

7. Intrauterine device according to one of the preceding claims, **characterised in that** the copper powder (P) is a pure copper powder, of a purity equal to or greater than 99.9 % and of a particle size less than or equal to 10 µm.

8. Intrauterine device according to one of the preceding claims, **characterised in that** a progestogen hormone is incorporated in the material (M).

9. Intrauterine device according to one of the preceding claims, **characterised in that** the material (M) is covered with a porous membrane adapted to allow regular and controlled release of the active ingredient (P).
